# EUROPEAN PATENT APPLICATION

(11) **EP 4 681 891 A1**
(43) Date of publication of application: **21.01.2026**
(21) Application number: 23927584.5
(22) Date of filing: 09.11.2023
(51) Int. Cl.: B25J 13/00, B25J 5/00, C12M 1/00, G01N 35/00, G01N 35/02

(54) **WORK INSTRUCTION DEVICE**

(30) Priority: 15.03.2023 JP 2023041385
(71) Applicant: OMRON Corporation, Kyoto-shi, Kyoto 600-8530 (JP); CHUGAI SEIYAKU KABUSHIKI KAISHA, Tokyo 115-8543 (JP)
(72) Inventor: SHIBATA, Yoshiya, Kyoto-shi, Kyoto 600-8530 (JP); NAKASHIMA, Chisato, Kyoto-shi, Kyoto 600-8530 (JP); HASHIMOTO, Takuma, Kyoto-shi, Kyoto 600-8530 (JP); YASE, Satoshi, Kyoto-shi, Kyoto 600-8530 (JP); KITAJIMA, Hiroshi, Kyoto-shi, Kyoto 600-8530 (JP); KURISU, Takanori, Kyoto-shi, Kyoto 600-8530 (JP)
(74) Representative: SONN Patentanwälte GmbH & Co KG
(86) International application number: PCT/JP2023/040474
(87) International publication number: WO 2024/189973

(57) **Abstract**

A robot system (100) including mobile manipulators (10) each including a local controller (11), workbench robots (20) each including a local controller (21), an integration controller (30), and a work instruction device (40) that performs a work instruction to execute an experiment on cells. The work instruction device (40) accesses experiment definition data including preparation transport information and workbench experiment specifying information, and creates a preparation transport instruction to take cells that are a target of the experiment out from a first storage location and transport them to a workbench based on the preparation transport information, and a workbench experiment instruction for executing the experiment on the transported cells based on the workbench experiment specifying information.

## Description

### Technical Field

The present disclosure relates to a work instruction device that creates a work instruction to control a robot.

### Background Art

In the fields of biochemistry, biology, and biotechnology, computer technology exists to create a protocol for an experiment using a notation method employing process symbols. For example, a proposed protocol chart creation device (see Patent Document 1) includes an initial symbol arrangement unit configured to arrange an initial symbol expressing an initial state of a container for holding a specimen, a procedure line arrangement unit configured to arrange a procedure line representing a process order for the container in a direction along a first axis from the initial symbol, a process symbol arrangement unit that is configured to arrange processing symbols representing processing to be carried out on the container along the procedure line and that is a process symbol arrangement unit to, in cases in which there are plural processes to be carried out on a single container, arrange the process symbols representing the processes along the procedure line, and a separation unit configured to separate arrangements of the initial symbols, the procedure lines, and the process symbols for different containers in a direction along a second axis intersecting the first axis.

Technology also exists to execute a protocol created by a computer with a robot. For example, there is a proposal for a system to automatically generate an operation command that causes a processing system including a robot to perform an experiment based on a protocol (Patent Document 2). Herein, an operation command generation device includes a processing job generation unit that generates one or more job corresponding to two or more processing symbols based on plural process symbols having a determined processing sequence and based on one or more job stored in a job storage unit, and a connecting job generation unit that, for two process symbols consecutive in processing sequence and based on a first reference point associated with a first job to be performed last out of the one or more jobs corresponding to the process symbols and having an earlier processing sequence number and based on a second reference point associated with a second job later in the processing sequence and performed first from out of the one or more jobs corresponding to the process symbols, generates a connecting job to move an arm from the first reference point to the second reference point.

### Related Art

### Patent Documents

Patent Document 1: Japanese Patent Publication No. 5886482
Patent Document 2: Japanese Patent Application No. 6399214

### SUMMARY OF INVENTION

### Technical Problem

Generally, an experiment protocol is one listing procedures of experiment manipulations performed by a researcher on a workbench and, in the technologies described in Patent Document 1 and Patent Document 2, a computer is utilized to create such a protocol, and the experiment manipulations are performed by a robot based on the protocol.

However, in relation to experiments handling cells, there are often situations in which cells processed in experiment manipulations on a workbench are stored or cultured in an incubator or the like, and after taking out specific cells that have been stored or cultured, experiment manipulations are performed on these cells in a follow-on experiment.

An object of the present disclosure is to automate an experiment handling cells that includes taking cells that are the target of the experiment out and, as required, storing cells after processing has been performed on them by experiment manipulations.

### Solution to Problem

In order to achieve the above objective, a work instruction device according to the present disclosure is, for a robot system including a mobile manipulator equipped with a mechanism for moving over a floor and a mechanism for gripping an object, a workbench robot that is fixed to a workbench and equipped with a mechanism for gripping an object on the workbench, and a controller for controlling operation of the mobile manipulator and a controller for controlling operation of the workbench robot, a work instruction device that performs a work instruction to execute an experiment on cells. The work instruction device includes an experiment definition data access section that accesses experiment definition data including preparation transport information that is information needed to take the cells that are a target of the experiment out from a first storage location and workbench experiment specifying information that is information to specify an experiment by the workbench robot, and an instruction creation section that creates a preparation transport instruction for the controller to control operation of the mobile manipulator and a workbench experiment instruction for the controller to control operation of the workbench robot. The preparation transport instruction is an instruction created based on the preparation transport information to take the cells that are the experiment target out from the first storage location and transport them to the workbench, and the workbench experiment instruction is an instruction created based on the workbench experiment specifying information to execute the experiment on the transported cells.

### Advantageous Effects

The work instruction device according to the present disclosure is able to automate an experiment handling cells that includes taking out cells that are the target of an experiment and, as required, storing cells after being processed by experiment manipulations.

### BRIEF DESCRIPTION OF DRAWINGS

Fig. 1 is a schematic plan view illustrating an example of an experiment environment for running a robot system according to the present exemplary embodiment.
Fig. 2 is a block diagram illustrating a schematic configuration of a robot system according to the present exemplary embodiment.
Fig. 3 is a perspective view of the external appearance of a mobile manipulator.
Fig. 4 is a perspective view of the external appearance of a workbench robot.
Fig. 5 is a block diagram illustrating a hardware configuration of a work instruction device.
Fig. 6 is a block diagram illustrating an example of a functional configuration of a work instruction device.
Fig. 7 is a flowchart illustrating a flow of work instruction processing for a case in which a thaw experiment is performed.
Fig. 8 is a diagram illustrating an example of thaw experiment pre-input data.
Fig. 9 is a diagram illustrating an example to thaw experiment start-time data.
Fig. 10 is a diagram illustrating an example of follow-on experiment data for a thaw experiment.
Fig. 11 is a flowchart illustrating a flow of work instruction processing for a case in which a maintain subculturing experiment is performed.
Fig. 12 is a diagram illustrating an example of maintenance subculturing experiment pre-input data.
Fig. 13 is a diagram illustrating an example of maintenance subculturing experiment start-time data.
Fig. 14 is a diagram illustrating an example of maintenance subculturing protocol data.
Fig. 15 is a diagram illustrating an example of follow-on experiment data in a maintenance subculturing experiment.
Fig. 16 is a diagram illustrating an example of expansion subculturing experiment start-time data.
Fig. 17 is a diagram illustrating an example of follow-on experiment data in an expansion subculturing experiment.
Fig. 18 is a flowchart illustrating a flow of work instruction processing in a case in which an assay pre-processing experiment is performed.
Fig. 19 is a diagram illustrating an example of assay pre-processing experiment pre-input data.
Fig. 20 is a diagram illustrating an example of assay pre-processing experiment start-time data.
Fig. 21 is a diagram illustrating an example of follow-on experiment data in an assay pre-processing experiment.
Fig. 22 is a flowchart illustrating a flow of control processing by an integration controller.
Fig. 23 is a flowchart illustrating a flow of subculturing.
Fig. 24 is a diagram to explain seeding of cells in a second container.
Fig. 25A is a diagram to explain another example of a controller configuration.
Fig. 25B is a diagram to explain another example of a controller configuration.
Fig. 26A is a diagram to explain another example of a controller configuration.
Fig. 26B is a diagram to explain another example of a controller configuration.

### DESCRIPTION OF EMBODIMENTS

Description follows regarding an example of an exemplary embodiment of the present disclosure, with reference to the drawings. Note that the same reference numerals will be appended across the drawings to the same or equivalent configuration elements and parts. Moreover, dimensions and proportions in the drawings are exaggerated for ease of explanation, and sometimes differ from actual proportions.

In the present exemplary embodiment, a robot system will be described that automates experiments handling cells in an environment similar to an experiment environment utilized by a researcher. Note that reference to "an experiment" in the present exemplary embodiment is not limited to something performed with an objective of finding out a result of manipulation or processing on a target such as cells or the like, and encompasses manipulation or processing on a target without investigating a result thereof. For example, subculturing of cells is also called an experiment.

A typical example of an experiment environment utilized by a researcher is an environment installed with a workbench to perform most experiment work, as well as locations somewhere away from the workbench installed with a cell observation device, a cell measurement device, a centrifuge, and the like. When a researcher performs experiment work, the researcher performs experiment work at the workbench, and also, as required during performing the experiment work, moves between the workbench and the cell observation device, the cell measurement device, the centrifuge, or the like by walking carrying a specimen container in order to use these devices.

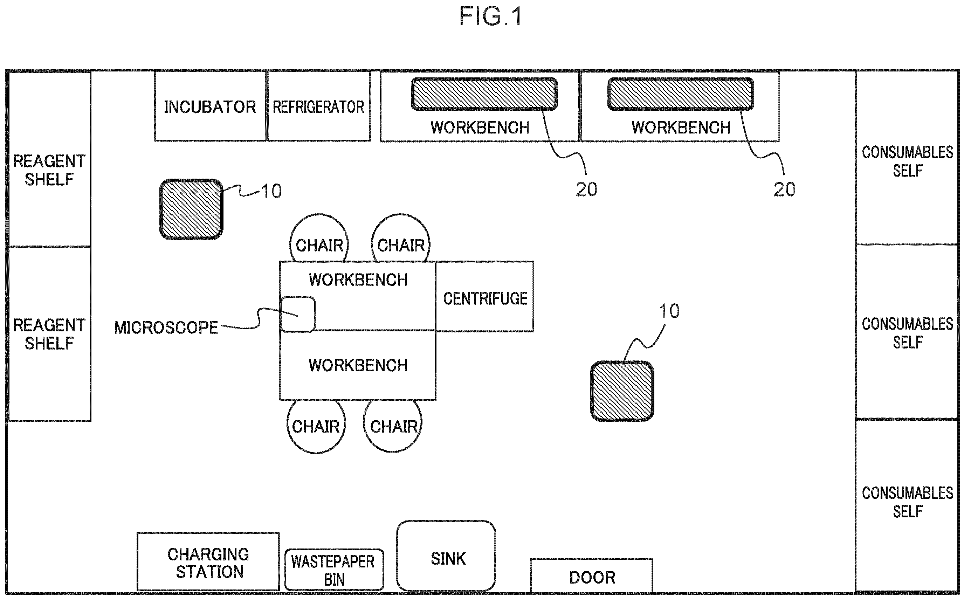

The robot system according to the present exemplary embodiment runs in an environment similar to the typical example of an experiment environment utilized by a researcher as described above. Fig. 1 is a schematic plan view illustrating an example of an experiment environment for running a robot system according to the present exemplary embodiment. In the example of Fig. 1, in addition to the above cell observation device, cell measurement device, centrifuge, and workbench, the experiment environment also includes experiment equipment such as reagent shelves, an incubator, a refrigerator, consumables shelves, a charging station, a sink, chairs, and the like. Note that a microscope in Fig. 1 is an example of the cell observation device and the cell measurement device referred to above.

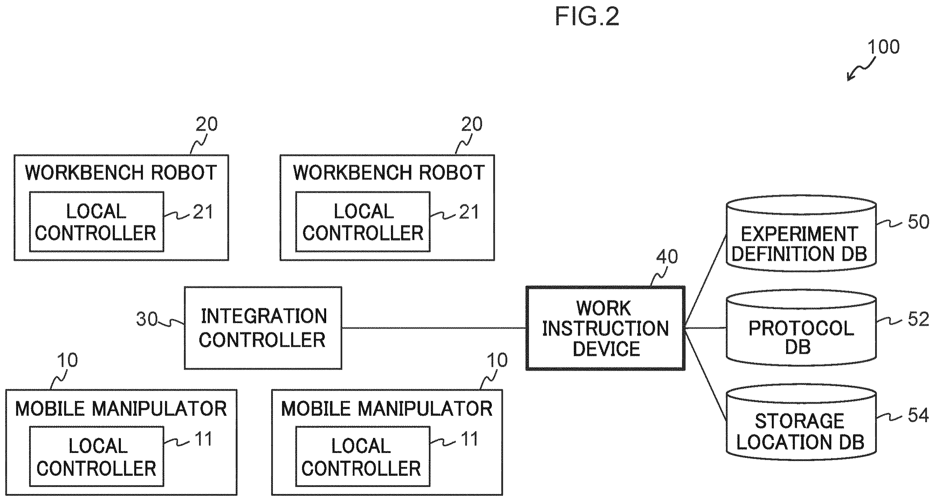

As illustrated in Fig. 2, a robot system 100 according to the present exemplary embodiment includes mobile manipulators 10, workbench robots 20, an integration controller 30, a work instruction device 40, an experiment definition database (DB) 50, a protocol DB 52, and a storage location DB 54. Note that although there are two mobile manipulators 10 and two workbench robots 20 illustrated in the example of Fig. 2, there is no limitation thereto. There may be a single, or three or more, of the mobile manipulator 10 and/or the workbench robot 20.

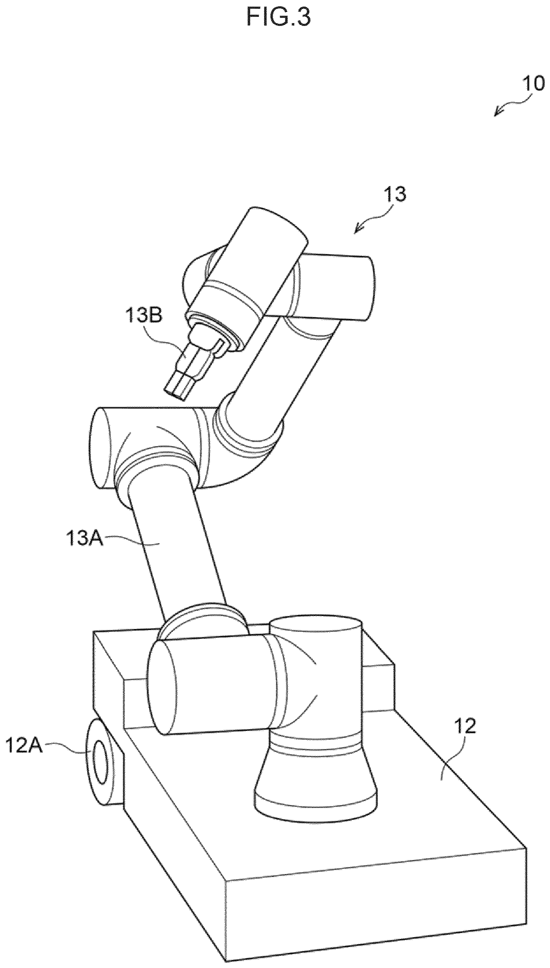

The mobile manipulators 10 each include a local controller 21, a moving mechanism to move across the floor, and a gripping mechanism to grip an object. Fig. 3 is a perspective view of the external appearance of the mobile manipulator 10. As illustrated in Fig. 3, the mobile manipulator 10 includes a trolley 12 and a robot arm 13.

The trolley 12 is an example of a moving mechanism, and includes two drive wheels 12A driven under control of a local controller 11. The trolley 12 also includes variable direction casters on a bottom face at the nearside in Fig. 3. The trolley 12 is able to move freely over the floor similarly to a researcher by the two drive wheels 12A being driven independently from each other. The moving mechanism is not limited to two independently driven wheels, and may be any freely selected mechanism capable of moving over the floor, and is preferably a mechanism having few limitations in the way of moving including turning. The robot arm 13 is an example of a gripping mechanism and is mounted on the trolley 12. The robot arm 13 includes an arm 13A, and a hand 13B attached to a distal end of the arm 13A. The arm 13A may be an articulated arm provided with a configuration to change the position and pose of the hand 13B in three dimensional space with, for example, six degrees of freedom. The hand 13B is, for example, a two fingered robot hand capable of gripping a container or the like. Note that although the mobile manipulator 10 illustrated in Fig. 3 is an example equipped with a single robot arm 13, a two handed configuration equipped with two robot arms 13 may be adopted.

The mobile manipulator 10 includes a non-illustrated vision sensor at a site near to the hand 13B of the arm 13A. The vision sensor is a sensor for recognizing objects in the periphery of the mobile manipulator 10, and gripping target objects for gripping with the hand 13B, such as a container or the like. The vision sensor is configured including a camera and an image processing device, and recognizes peripheral objects and gripping target objects by using the image processing device to perform image processing on images captured with the camera. The mobile manipulator 10 is accordingly able to move autonomously. Note that the sensor for recognizing peripheral objects and gripping target objects is not limited to being a vision sensor, and a laser radar or the like capable of measuring a three-dimensional position of each point in the periphery may be employed. A sensor for recognizing peripheral objects may also be mounted to the trolley 12.

The local controller 11 controls operation of each motor of the mobile manipulator 10 so as to be able to implement instructions of the integration controller 30. Specifically, the local controller 11 controls the operation of the mobile manipulator 10 based on recognition results output from the vision sensor, instructions from the integration controller 30, and the like. More specifically, the local controller 11 controls the operation of the mobile manipulator 10 so as to grip a gripping target object such as a container or the like with the hand 13B, and to transport the gripping target object that has been gripped to a specific position, such as the workbench or the like.

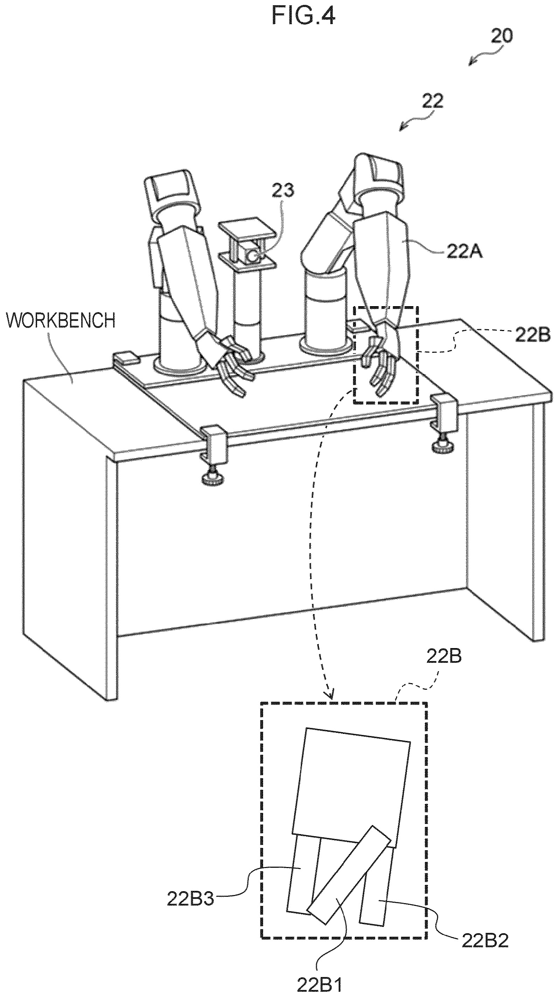

The workbench robots 20 are each fixed to a workbench, and equipped with the local controller 21, and a gripping mechanism for gripping objects on the workbench. Gripping target objects gripped by the workbench robot 20 include, as well as containers, an aspirator, an electronic pipette, an electronic micro pipette, or the like for suctioning cells and the like inside a container or for discharging cells or the like to inside the container. Fig. 4 is a perspective view of the external appearance of the workbench robot 20. As illustrated in Fig. 4, the workbench robot 20 is equipped with two robot arms 22, and a vision sensor 23.

The robot arms 22 are each an example of a gripping mechanism, and include an arm 22A, and a hand 22B attached to a distal end of the arm 22A. The arm 22A may be an articulated arm including a configuration to change the position and pose of the hand 22B in three-dimensional space with, for example, six degrees of freedom. The hand 22B is, for example, a three-fingered robot hand capable of gripping a container or the like and including joints on each finger.

An expanded schematic diagram of the hand 22B is illustrated at the bottom of Fig. 4. In this schematic diagram the joints of each of the fingers of the hand 22B are omitted from illustration, and a simplified view of the shape and arrangement of each of the fingers is illustrated. The hand 22B includes a first group of fingers including a first finger 22B1, and a second group of fingers including a second finger 22B2 and a third finger 22B3, and is a structure capable of gripping an object between the opposing first group and second group. Corresponding to human fingers, the first finger 22B 1 works as a thumb, the second finger 22B2 works as an index finger, and the third finger 22B3 works as a middle finger. Note that the hand 22B may be a robot hand with four or more fingers. Namely, the first group may include a finger other than the first finger 22B1, and/or the second group may include a finger other than the second finger 22B2 and the third finger 22B3. Each of the fingers of the hand 22B preferably includes two joints partway along. This thereby enables wrapping around so as to grip the external shape of an object stably, and facilitates fine manipulation such as pressing a button or an experiment instrument or the like.

In the following the two hands 22B of the robot arms 22 will be denoted as a left hand 22BL and a right hand 22BR when discriminating in the description between left and right hands. Similarly for the fingers of the hand 22B, the fingers of the left hand 22BL will be denoted as first finger 22B1L, second finger 22B2L, and third finger 22B3L, and the fingers of the right hand 22BR will be denoted as first finger 22B1R, second finger 22B2R, and third finger 22B3R.

The vision sensor 23 is mounted to a pan tilt mount, and is a sensor for recognizing gripping target objects such as a container or the like arranged on the workbench.

The local controller 21 controls the operation of each motor of the workbench robot 20 so as to enable implementation of an instruction from the integration controller 30. Specifically, the local controller 21 controls operation of the workbench robot 20 based on recognition results output from the vision sensor 23, and instructions and the like from the integration controller 30. More specifically, the local controller 21 controls operation of the workbench robot 20 so as to execute experiment processes in which cells are handled by coordinating the two robot arms 22. For example, the local controller 21 controls operation of the workbench robot 20 so as to grip a container with the hand 22B of one of the robot arms 22, to grip a pipette with the hand 22B of the other robot arm 22, and to execute processes such as suctioning liquid inside the container.

Note that although in Fig. 4 an example is illustrated in which the workbench robot 20 is fixed directly to the workbench, the workbench robot 20 may have a fixed relative positional relationship to the workbench by, for example, being fixed to the floor, a wall, the ceiling, or the like.

Based on a work instruction from the work instruction device 40, the integration controller 30 interlinks the local controllers 11 of the mobile manipulators 10 and the local controllers 21 of the workbench robots 20 so as to control each operation of the mobile manipulator 10 and the workbench robots 20. Specifically, the integration controller 30 executes an experiment with the mobile manipulators 10 and the workbench robots 20 including transporting and placing a container containing cells between the workbench and other experiment equipment.

Gripping operations and manipulations of experiment equipment by each of the mobile manipulators 10 are implemented by the local controller 11 executing a pre-taught operation plan with the mobile manipulator 10 based on recognition results output from the vision sensor. Similar applies to gripping operations and manipulations of experiment equipment by the workbench robots 20.

Moreover, movement of each of the mobile manipulators 10 is implemented by the local controller 11 moving the mobile manipulator 10 to a position of experiment equipment as instructed by the integration controller 30 based on a pre-stored layout map of the experiment environment. Note that the local controller 11 moves the mobile manipulator 10 such that movement to a goal position is performed while avoiding obstacles based on the recognition results output from the vision sensor.

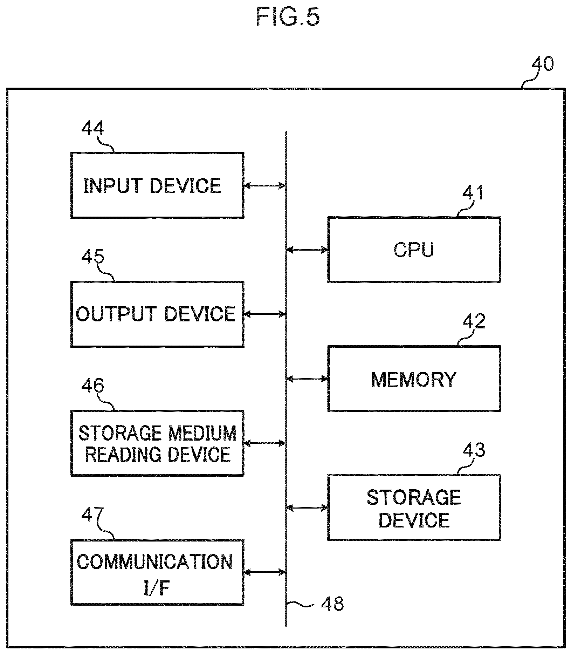

The work instruction device 40 performs work instructions to execute experiments on cells. Fig. 5 is a block diagram illustrating a hardware configuration of the work instruction device 40. As illustrated in Fig. 5, the work instruction device 40 includes a central processing unit (CPU) 41, memory 42, a storage device 43, an input device 44, an output device 45, a storage medium reading device 46, and a communication interface (I/F) 47. These configurations are connected together through a bus 48 so as to be capable of communicating with each other.

A program for executing work instruction processing, described later, is stored in the storage device 43. The CPU 41 is a central processing unit that executes various programs and controls each configuration. Namely, the CPU 41 reads a program from the storage device 43 and executes the program using the memory 42 as a work area. The CPU 41 controls the above configurations and performs various computation processing according to the program stored on the storage device 43.

The memory 42 is configured by random access memory (RAM), and is employed as a work area to temporarily store programs and data. The storage device 43 is configured by read only memory (ROM), and a hard disk drive (HDD), a solid state drive (SSD), or the like, and various programs including an operating system and various data are stored thereon.

The input device 44 is a device for performing various inputs and is, for example, a keyboard, a mouse, or the like. The output device 45 is a device for outputting various information and is, for example, a display, a printer, or the like. The output device 45 may also function as the input device 44 by utilizing a touch panel display therefor.

The storage medium reading device 46 performs reading of data stored on various storage mediums, such as compact disk (CD)-ROM, digital versatile disc (DVD)-ROM, Blu-ray disc, universal serial bus (USB) memory, or the like, and performs writing of data to these storage mediums. The communication I/F 47 is an interface for communication with other devices, and employs a standard such as, for example, Ethernet (registered trademark), FDDI, Wi-Fi (registered trademark), or the like. Note that other devices include the integration controller 30. Moreover, the other devices also include, as necessary, the local controller 11 of the mobile manipulator 10, the local controller 21 of the workbench robot 20, the cell observation device, the cell measurement device, the centrifuge, and the like. In the present exemplary embodiment these other devices are also equipped with a communication function.

Note that the hardware configurations of the integration controller 30, the local controller 11 of the mobile manipulator 10, and the local controller 21 of the workbench robot 20 are each substantially similar to the hardware configuration of the work instruction device 40, and so explanation thereof will be omitted.

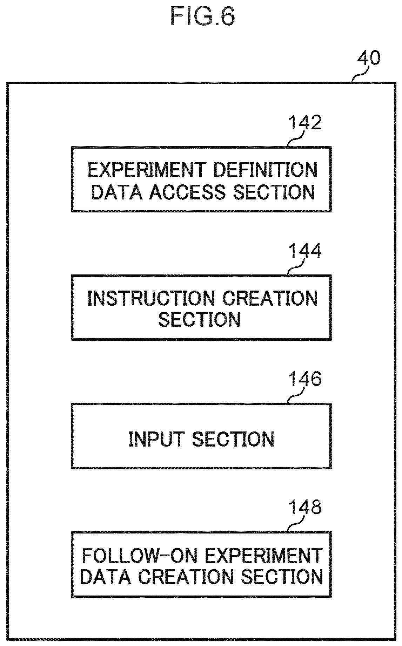

Next, description follows regarding a functional configuration of the work instruction device 40. Fig. 6 is a block diagram illustrating an example of a functional configuration of the work instruction device 40. As illustrated in Fig. 6, the work instruction device 40 includes, as functional configuration, an experiment definition data access section 142, an instruction creation section 144, an input section 146, and a follow-on experiment data creation section 148. Each of the functional configuration is implemented by the CPU 41 reading a work instruction program stored on the storage device 43 and expanding and executing the work instruction program in the memory 42.

The experiment definition data access section 142 accesses the experiment definition DB 50 that stores experiment definition data including preparation transport information, workbench experiment specifying information, and storage transport information. The physical location of the experiment definition DB 50 may be freely selected as long as it is accessible by the work instruction device 40. For example, the experiment definition DB 50 may be stored in the storage device 43 inside the work instruction device 40, may be stored in an external storage device connected to the work instruction device 40, or may be stored on a server accessible over a network. For example, an indirect access format may be employed in which the work instruction device 40 designates and requests experiment definition data from an external device identified by data configured by a key identifying an experiment such as an experiment ID such that, in response to this request, the experiment definition data instructed is sent to the work instruction device 40.

The preparation transport information is information needed to take cells that are the experiment target out from a first storage location. The workbench experiment specifying information is information to specify an experiment by the workbench robot 20. The storage transport information is information for transporting the cells to a second storage location after the experiment has been performed at the workbench.

Note that the experiment specified by the workbench experiment specifying information is not limited to an experiment in which manipulation or processing is performed by the workbench robot 20 alone, and may be an experiment in which the mobile manipulator 10 is involved partway through the experiment. Parts involving the mobile manipulator 10 includes an instruction to a controller to control the operation of the mobile manipulator 10 in a workbench experiment instruction. Reference here to "controller" means a controller that can be given a workbench experiment instruction from the work instruction device 40, and corresponds to the integration controller 30 in the exemplary embodiment of Fig. 2. The operation of the mobile manipulator 10 is controlled stepwise by the integration controller 30 and the local controller 11, and so the integration controller 30 also corresponds to a controller to control operations of the mobile manipulator 10. A manner of involvement of the mobile manipulator 10 partway through an experiment is, for example, by a reagent, an instrument, or the like being transported by the mobile manipulator 10, or by experiment target cells being transported by the mobile manipulator 10 between the workbench and a device such as a cell observation device, centrifuge, incubator, or the like. The incubator referred to here is not one whose main objective is to culture cells and, for example, is envisaged as being utilized in assay pre-processing to place cells for a fixed time in an environment at a constant temperature after a reagent has been administered to the cells in order to wait for an effect of the reagent on the cells to appear.

Moreover, the experiment definition data access section 142 stores, in the experiment definition DB 50, follow-on experiment data created by the follow-on experiment data creation section 148, described later.

The instruction creation section 144 creates a preparation transport instruction for the controller to control operation of the mobile manipulators 10, and a workbench experiment instruction for the controller to control operation of the workbench robots 20. The "controller" referred to here is a controller that can be given the preparation transport instruction or workbench experiment instruction from the work instruction device 40, and so corresponds to the integration controller 30 in the exemplary embodiment of Fig. 2. The operation of the mobile manipulator 10 is controlled stepwise by the integration controller 30 and the local controller 11, and so the integration controller 30 also corresponds to a controller to control operation of the mobile manipulator 10. Moreover, the operation of the workbench robot 20 is controlled stepwise by the integration controller 30 and the local controller 21, and so the integration controller 30 also corresponds to a controller to control operation of the workbench robot 20.

The preparation transport instruction is an instruction created based on the preparation transport information for taking cells that are the experiment target from the first storage location and transporting them to the workbench. For example, the preparation transport instruction includes an instruction related to movement of the mobile manipulator 10, and an instruction related to manipulation of the mobile manipulator 10. The instruction related to movement of the mobile manipulator 10 includes, for example, information about a position of the movement destination. The instruction related to manipulation of the mobile manipulator 10 includes, for example, information for gripping a target object, and information for placing the gripped target object. The information for gripping the target object includes, for example, information specifying a container and information specifying a location of the container when the gripping target object is a container. The information for placing the target object includes, for example, information of a position on the workbench for placing the target object.

The workbench experiment instruction is created based on the workbench experiment specifying information and is an instruction to execute an experiment on transported cells.

The instruction creation section 144 creates a storage transport instruction for a controller to control operation of the mobile manipulator 10. The storage transport instruction is created based on the storage transport information, and is an instruction to transport the cells to the second storage location after the experiment has been performed on the workbench. The reference here to "controller" means a controller that that can be given the storage transport instruction from the work instruction device 40, and so this applies to the integration controller 30 in the exemplary embodiment of Fig. 2. The operation of the mobile manipulator 10 is controlled stepwise by the integration controller 30 and the local controller 11, and so the integration controller 30 also corresponds to the controller for controlling operation of the mobile manipulator 10.

The input section 146 receives input of the workbench experiment specifying information. In cases in which the workbench experiment specifying information is not contained in the experiment definition data corresponding to the experiment that is expected to be executed, or in cases in which there is a desire to change the workbench experiment specifying information contained in this experiment definition data, the input section 146 receives the workbench experiment specifying information input from a user.

The follow-on experiment data creation section 148 creates data for a follow-on experiment containing data specifying a container holding cells to be utilized in the follow-on experiment or data specifying a second storage location for storing a container holding the cells (hereafter referred to as "follow-on experiment data").

The follow-on experiment data may include both the data specifying the container holding the cells and also data specifying the second storage location for storing the container holding the cells, or may include one of these types of data. Management is performed so as to be able to identify the second storage location from data specifying the container in cases in which data of the second storage location is not contained. For example, which container has been received in which storage location may be recoded in a separately prepared system for managing storage locations at the point in time when a container has been received at a second storage location. This thereby enables a storage location of such a container to be found from information specifying the container when a follow-on experiment is to be performed.

Moreover, a container stored with post-experiment cells may be supplied to the follow-on experiment by being placed in a second storage location within the reach of a hand of the workbench robot 20 without being transported by the mobile manipulator 10. There is no need to perform preparation transport using the mobile manipulator 10 in the follow-on experiment as long as the follow-on experiment is to be performed at the same workbench.

Next, description follows regarding the operation and advantageous effects of the robot control system 100 according to the present exemplary embodiment. Description follows of respective cases in which the experiment is a thaw experiment, a subculturing experiment, and an assay pre-processing experiment. Note that in the present exemplary embodiment, the objective of subculturing is to continue the presence of the cells, and this may be so-called maintenance subculturing that is subculturing without the objective of increasing the number of cells, and may be so-called expansion subculturing that is subculturing with an objective of increasing the number of cells. Although the number of cells being cultured in an incubator also increases during maintenance subculturing, the number of cells for seeding in a new container in a subculturing experiment is about the same as the number of cells prior to culturing. Cells not seeded in the new container are discarded. For cases of expansion subculturing, a greater number of cells are seeded in new container(s) than the number of cells prior to culturing. This means that in such cases plural containers or a larger container are/is employed as the new container(s) for seeding the cultured cells that were inside a single cell container prior to a subculturing experiment.

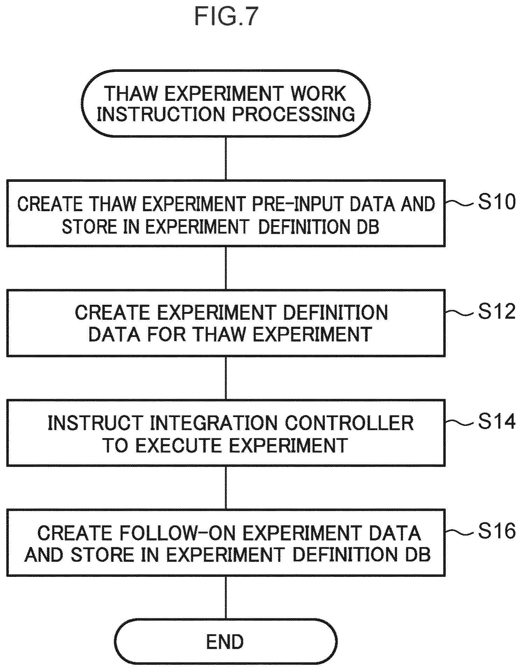

Fig. 7 is a flowchart illustrating a flow of work instruction processing for a case in which a thaw experiment is performed by the CPU 41 of the work instruction device 40.

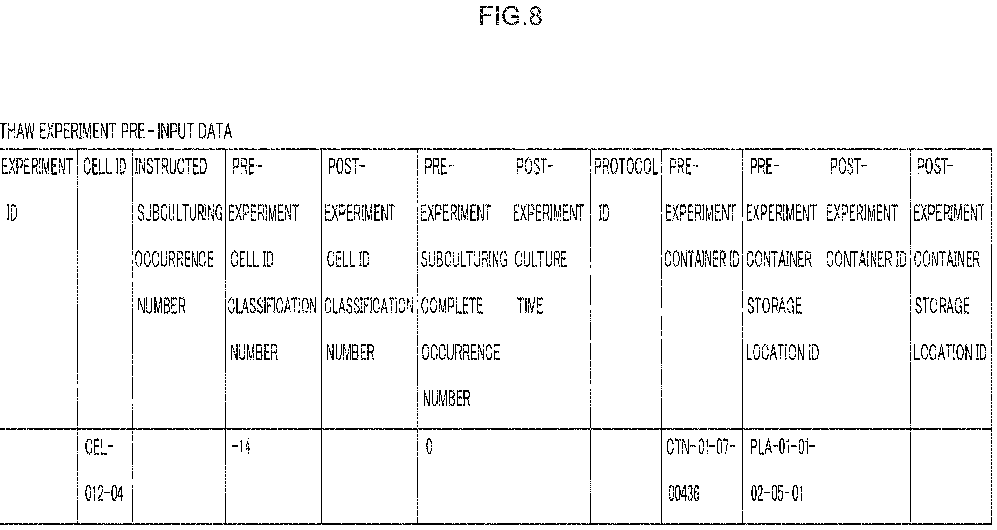

At step S10, when thawing the cells, the instruction creation section 144 creates thaw experiment pre-input data including data of a cell ID, a cell ID classification number, a container ID, and a container storage location ID. The container ID and the container storage location ID are examples of preparation transport information. The experiment definition data access section 142 stores the created thaw experiment pre-input data in the experiment definition DB 50. Fig. 8 illustrates an example of the thaw experiment pre-input data stored in the experiment definition DB 50. Each ID may be appended automatically based on a predetermined rule, or input or amendment of each ID may be received manually through the input section 146.

In the example of Fig. 8, the notation format of the cell ID is "CEL-aaa-bb". CEL is a text string indicating that it is a cell ID, aaa is a number representing a type of cell, and bb is a number to discriminate an acquisition route, mutant strain, or the like. Moreover, the notation format of the cell ID classification number is "-n-o-p-...". n, o, p, ... are appended in sequence each time cells are divided into plural containers in expansion subculturing, so as to update the cell ID classification number with which number of container the cells are in when cells have been divided into plural containers. The example of Fig. 8 indicates n = 14, with this indicating that the cells that have been thaw processed are cells of the 14th container from out of cells prior to freezing that were divided into plural containers.

Moreover, the notation format of the container ID is "CTN-cc-dd-eeeee". CTN is a text string indicating that it is a container ID, cc is a number representing a course classification of container type and, for example, 01 is a conical tube, 02 is a square flask, and the like. dd is a number representing a fine classification of container type and, for example, is a number or the like associated with a container product number. eeee is an individual number of a container. The container ID may be acquired by reading an optical readable code such as a barcode, or a radio frequency identification (RFID) tag or the like attached to the container. A position of a container specified by the container ID may be tracked, such that the container ID is acquired from the current position of the container.

The notation format of the container storage location ID is "PLA-ff-gg-hh-ii-jj". PLA is a text string indicating that it is a container storage location ID, ff is a number representing a type of storage location and, for example, 01 is a refrigerator, 02 is an incubator, 03 is a storage shelf, 04 is a table, and the like. gg is a number to discriminate between storage locations of the same type and, for example, 03 is machine No. 3 (refrigerator No. 3 when ff is 01 refrigerator). hh is a number expressing an area in the storage location where the container rack is placed, a number expressing an area in the storage location where the container is placed when a container rack is not used, and, for example, is a shelf number. ii is a number expressing a position inside the area where a container rack is placed, or is a number expressing a position inside an area where the container is placed when a container rack is not used, and, for example, is a number representing a position on a shelf. jj is a number expressing a position of a container in a container rack, and, for example, is 00 when a container rack is not used.

Storage location data associated with the storage location ID and a position of the storage location are stored in the storage location DB 54. The storage location position is represented by a combination of a position of equipment such as a refrigerator, an incubator, a storage shelf, or a table that is the storage location, and a position inside the equipment. The position of the equipment is, for example, expressed by coordinates set in a room where the equipment is placed. The position inside the equipment is, for example, represented by coordinates set within each equipment. The physical location of the storage location DB 54 may be within the same storage device as the one where the experiment definition DB 50 is stored, or may be within a separate storage device. For example, the storage location DB 54 may be stored in a storage device inside the integration controller 30. In cases in which the storage location DB 54 is not stored inside the integration controller 30, the integration controller 30 may acquire the storage location data from a storage location DB via the work instruction device 40, or may, for example, acquire the storage location data directly from the storage location of the storage location DB 54 using wireless communication, without going through the work instruction device 40.

Next, at step S12, the instruction creation section 144 creates experiment definition data for a thaw experiment (thaw experiment start-time data). Specifically, the input section 146 receives input of either a cell ID (and cell ID classification number), or a pre-experiment container ID, or a pre-experiment container storage location ID. The instruction creation section 144 searches the experiment definition DB 50 with whichever ID was received, and acquires corresponding thaw experiment pre-input data from the experiment definition DB 50 as the experiment definition data. The instruction creation section 144 specifies a protocol ID of the thaw experiment. The protocol ID is an example of workbench experiment specifying information. The protocol ID may be specified by receiving an input, or may be specified by being selected from a list of displayed selection options. The instruction creation section 144 adds the specified protocol ID to the experiment definition data.

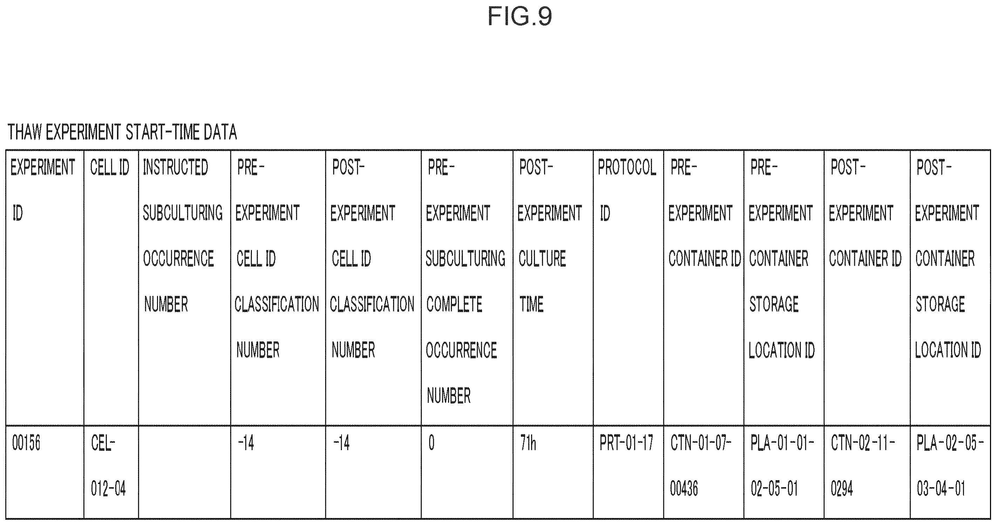

In the example of Fig. 9, the notation format of the protocol ID is "PRT-kk-lll". PRT is a text string indicating that it is a protocol ID. kk is a number expressing a type of protocol and, for example, 01 is thawing, 02 is maintenance subculturing, 03 is expansion subculturing, 04 is assay pre-processing, and the like. lll is an identification number within the same type of protocol.

The instruction creation section 144 adds an experiment ID, a post-experiment cell ID classification number, a post-experiment container ID, and a post-experiment container storage location ID to the experiment definition data. Each of the IDs may be appended automatically based on a predetermined rule, or input or amendment of each ID may be received manually through the input section 146.

Fig. 9 illustrates an example of experiment definition data (thaw experiment start-time data) that is created. This experiment definition data may be temporarily stored in the experiment definition DB 50, or may be configured so as to be transmitted to the integration controller 30 without being stored in the experiment definition DB 50. In the former case, the experiment definition data access section 142 stores the experiment definition data created by the instruction creation section 144 in the experiment definition DB 50.

Next, at step S14, the instruction creation section 144 instructs the integration controller 30 to execute an experiment. Specifically, the input section 146 receives input of an experiment ID. The instruction creation section 144 searches the experiment definition DB 50 with the received experiment ID and acquires the corresponding experiment definition data from the experiment definition DB 50. This processing is not needed in cases in which the experiment definition data created at step S12 is transmitted without being stored in the experiment definition DB 50. Note that instead of using the experiment ID, the experiment definition data may be searched using an ID from out of a cell ID (and cell ID classification number), a pre-experiment container ID, or a pre-experiment container storage location ID.

The instruction creation section 144 acquires the protocol data specified by the protocol ID contained in the acquired experiment definition data from the protocol DB 52. The protocol data is data including a protocol ID, an experiment content, target cells, an experiment procedure, and the like listed in text format. Plural protocol data is stored in the protocol DB 52 according to each experiment type. The instruction creation section 144 instructs execution of an experiment by transmitting a command instructing experiment execution, experiment definition data, and the protocol data to the integration controller 30.

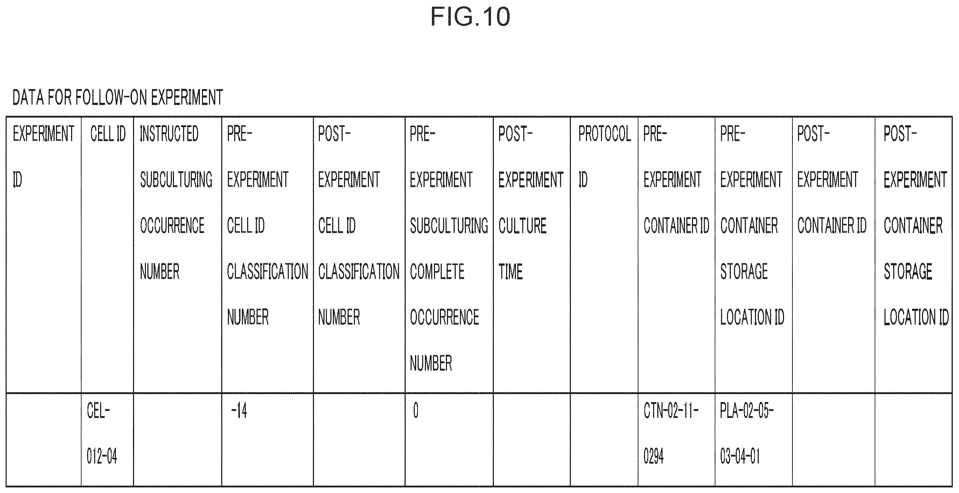

Next, at step S16, the instruction creation section 144 creates follow-on experiment data. Specifically, the instruction creation section 144 carries over the cell ID contained in the experiment definition data of the experiment for which execution was instructed, and also creates follow-on experiment data such as illustrated in Fig. 10, in which the post-experiment cell ID classification number, post-experiment container ID, and post-experiment container storage location ID contained in the experiment definition data of the experiment for which execution was instructed are employed as the pre-experiment cell ID classification number, the pre-experiment container ID, and the pre-experiment container storage location ID. Then the experiment definition data access section 142 stores the created follow-on experiment data in the experiment definition DB 50. The work instruction processing for performing a thaw experiment is then ended.

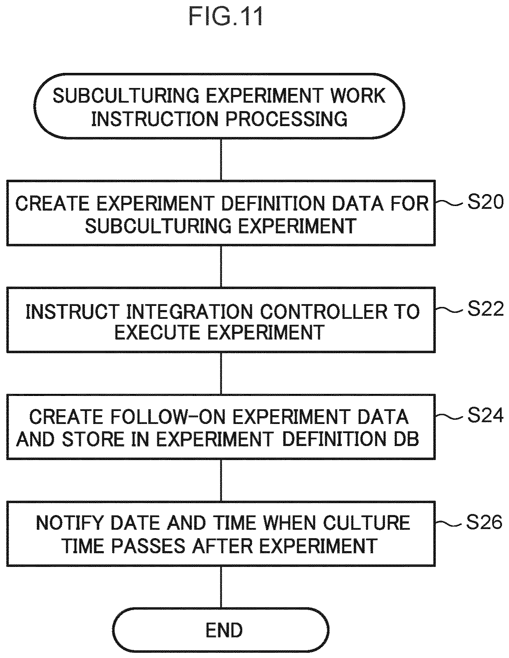

Fig. 11 is a flowchart illustrating a flow of work instruction processing for a case in which a maintenance subculturing experiment is executed by the CPU 41 of the work instruction device 40. Note that detailed explanation will be omitted for processing similar to the work instruction processing for when a thaw experiment is performed as illustrated in Fig. 7.

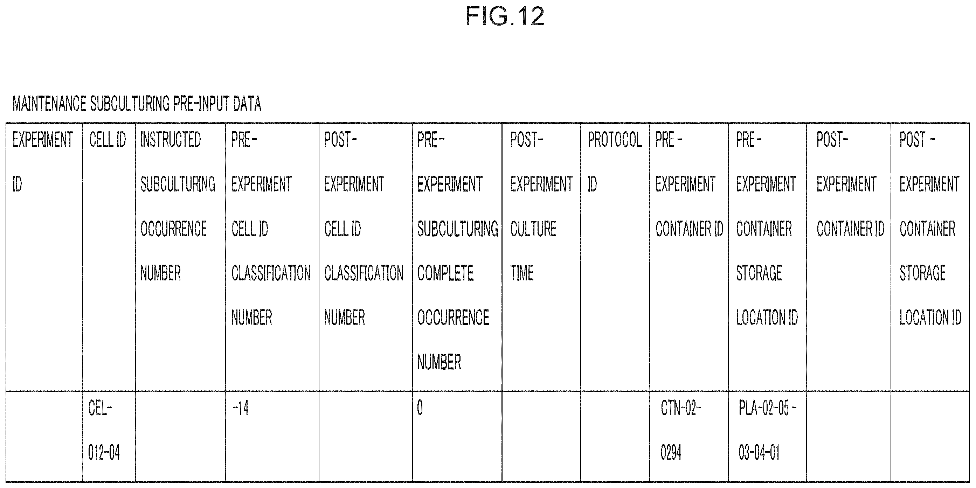

At step S20, the instruction creation section 144 creates experiment definition data for a maintenance subculturing experiment (maintenance subculturing experiment start-time data). Specifically, the experiment definition DB 50 is searched with an ID from out of a cell ID (and cell ID classification number), a pre-experiment container ID, and a pre-experiment container storage location ID, and corresponding maintenance subculturing experiment pre-input data is acquired as the experiment definition data from the experiment definition DB 50. As illustrated in Fig. 12, the maintenance subculturing experiment pre-input data is stored with follow-on experiment data for a past thaw experiment, maintenance subculturing experiment, or expansion subculturing experiment.

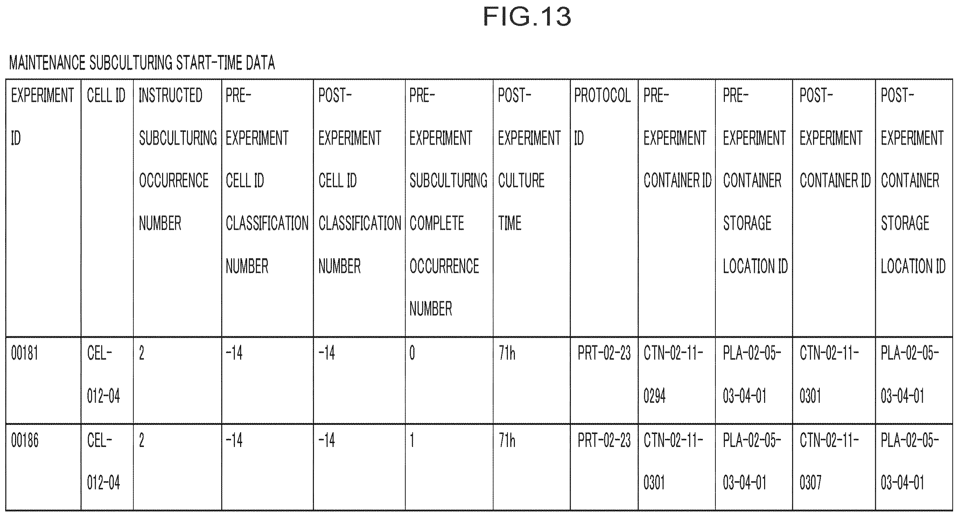

In cases in which the acquired experiment definition data is follow-on experiment data created by a past thaw experiment, the instruction creation section 144 specifies a protocol ID of a maintenance subculturing experiment and adds this to the experiment definition data, as illustrated in Fig. 13. Moreover, the instruction creation section 144 adds, to the experiment definition data, an experiment ID, an instructed subculturing occurrence number, a post-experiment cell ID classification number, a pre-experiment subculturing complete occurrence number, a post-experiment culture time, a post-experiment container ID, and a post-experiment container storage location ID.

In the example of Fig. 13, the notation format of the pre-experiment subculturing complete occurrence number is "m". m is incremented when performing subculturing irrespective of whether or not this is maintenance subculturing or expansion subculturing. Fig. 13 illustrates the subculturing complete occurrence number after thaw processing, however this subculturing occurrence number may be included for cases in which subculturing has been performed prior to thawing.

In cases in which the acquired experiment definition data is follow-on experiment data created by a past maintenance subculturing experiment or expansion subculturing experiment, the instruction creation section 144 adds, to the experiment definition data, the experiment ID, the post-experiment cell ID classification number, the post-experiment culture time, the post-experiment container ID, and the post-experiment container storage location ID. Each of the IDs may be appended automatically based on a predetermined rule, or input or amendment of each ID may be received manually through the input section 146. Moreover, the post-experiment culture time and the protocol ID may be changed manually.

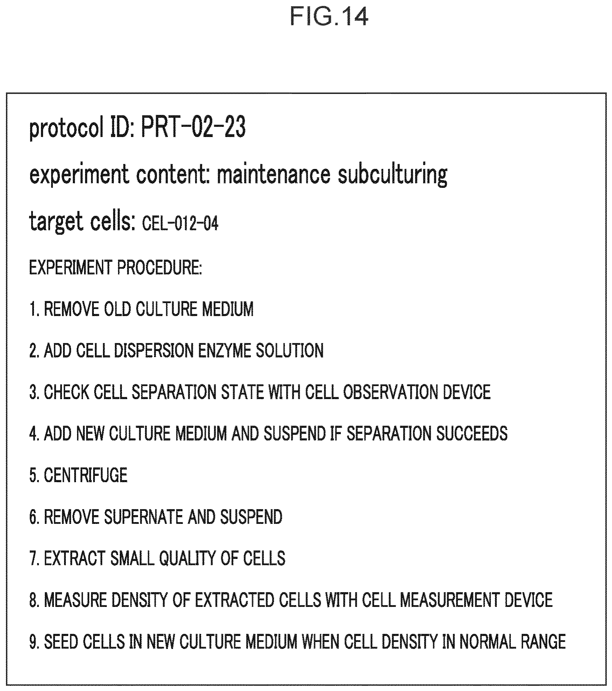

Next, at step S22, the instruction creation section 144 instructs the integration controller 30 to execute an experiment. The instruction creation section 144 searches the experiment definition DB 50 with the experiment ID, and acquires the corresponding experiment definition data from the experiment definition DB 50. The acquired experiment definition data is treated as maintenance subculturing experiment start-time data. The instruction creation section 144 acquires protocol data specified with the protocol ID contained in the acquired experiment definition data from the protocol DB 52. Fig. 14 illustrates an example of maintenance subculturing protocol data. Note that in an actual protocol data the type, quantity, etc. of reagents and culture media are also specified. The instruction creation section 144 instructs experiment execution by transmitting a command instructing experiment execution, the experiment definition data, and the protocol data to the integration controller 30.

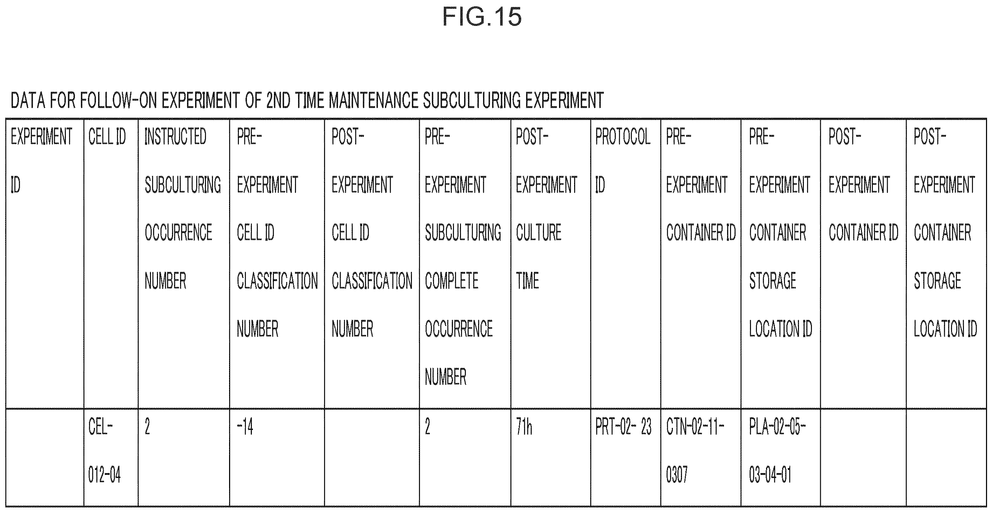

Next, at step S24, the instruction creation section 144 creates follow-on experiment data. Specifically, the instruction creation section 144 carries over the cell ID, instructed subculturing occurrence number, post-experiment culture time, and protocol ID of the experiment definition data of the experiment instructed for execution, and also creates follow-on experiment data that employs the post-experiment cell ID classification number, the post-experiment container ID, and the post-experiment container storage location ID of the experiment definition data for the experiment that was instructed for execution as the pre-experiment cell ID classification number, the pre-experiment container ID, and the pre-experiment container storage location ID. Moreover, the instruction creation section 144 also takes one occurrence added to the pre-experiment subculturing complete occurrence number of the experiment definition data that was instructed for experiment execution as the pre-experiment subculturing complete occurrence number of the follow-on experiment data. The experiment definition data access section 142 then, as illustrated in Fig. 15, stores the created follow-on experiment data in the experiment definition DB 50.

Next, at step S26, the instruction creation section 144 notifies a user of a date and time when a post-experiment culture time, contained in the experiment definition data of the experiment from the time point when experiment execution was instructed, passes. For example, the instruction creation section 144 may display a message may be on a screen, or may transmit a reminder e-mail to a user at a specific time prior to this date and time. Moreover, in cases in which the occurrence number of one occurrence added to the pre-experiment subculturing complete occurrence number contained in the experiment definition data of this experiment matches an instructed subculturing occurrence number, the instruction creation section 144 notifies that it is the date and time when the culture time after the final maintenance subculturing experiment passes. The work instruction processing for performing a maintenance subculturing experiment is then ended.

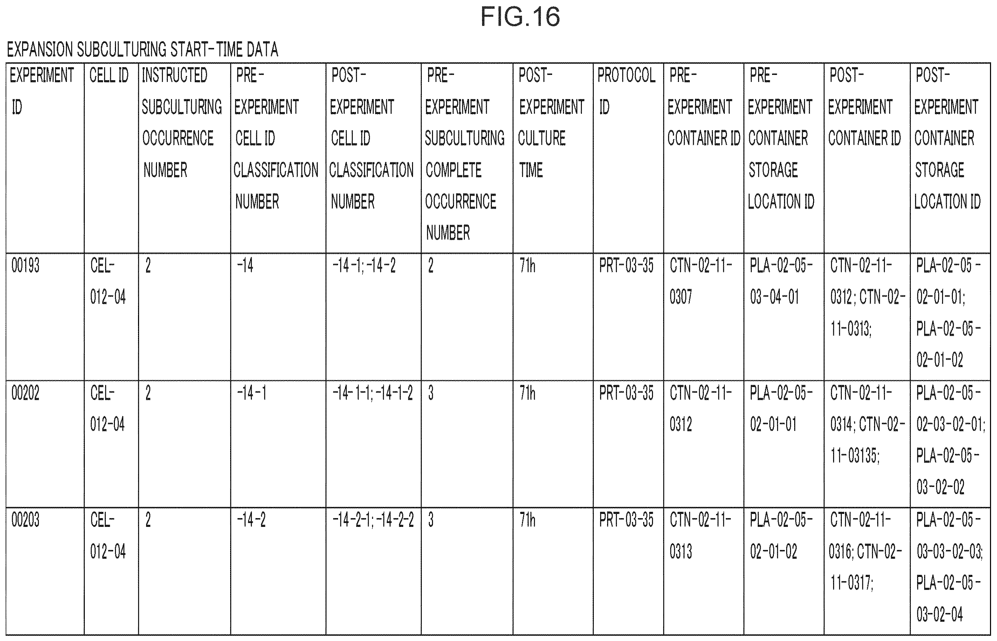

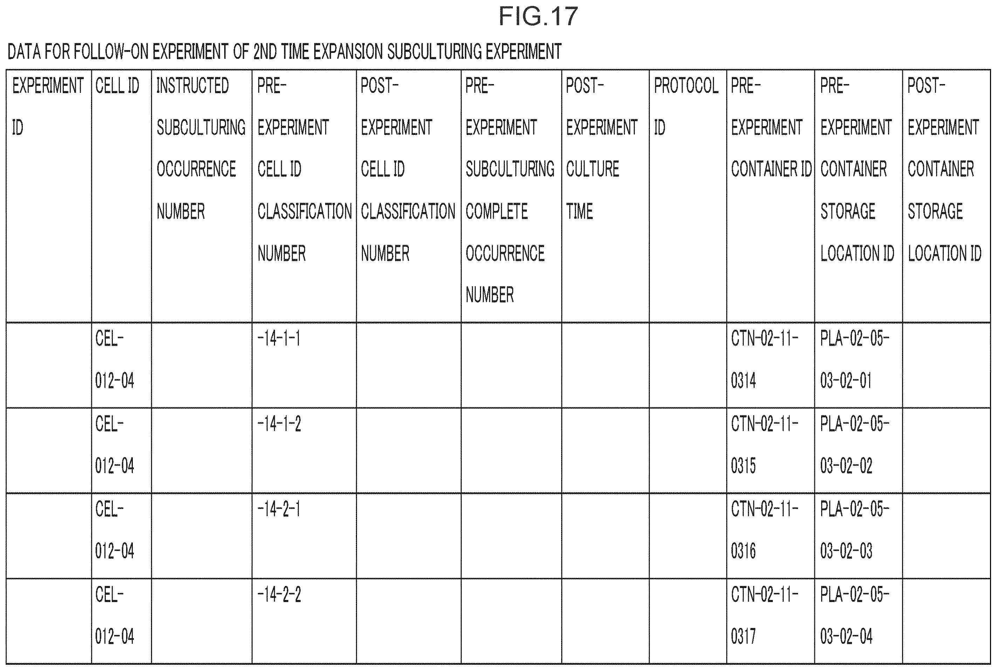

The work instruction processing for performing expansion subculturing experiment is similar to that of the work instruction processing for performing maintenance subculturing experiment as illustrated in Fig. 11. However, as illustrated in Fig. 16 and Fig. 17, it differs from a maintenance subculturing experiment in the point that there is also an increase in the experiment definition data and the follow-on experiment data corresponding to the increase in the number of containers in an expansion subculturing experiment.

Note that o = 1, 2 of the cell ID classification number (-n-o-p) in the example of Fig. 16 indicates cells that have been divided into a 1st container or a 2nd container in the expansion subculturing of experiment ID00193. Moreover, p = 1, 2 indicates cells that have been divided into a 1st container or a 2nd container in the expansion subculturing of experiment IDs 00202, 00203. For example, the post-experiment cell ID classification number "-14-2-1" of experiment ID00203 that is the 2nd expansion subculturing indicates cells put into the 2nd container in the 1st time of expansion subculturing, and cells put into the 1st container in the 2nd time of subculturing. This thereby enables the history of cell divisions to be tracked using the cell ID classification number.

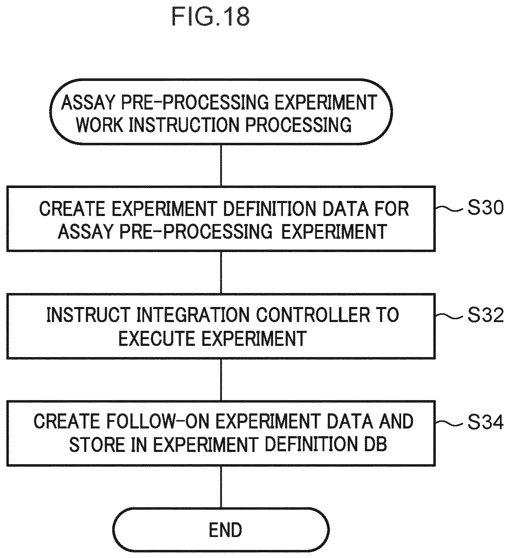

Fig. 18 is a flowchart illustrating a flow of work instruction processing for a case in which an assay pre-processing experiment is to be executed by the CPU 41 of the work instruction device 40. Note that detailed explanation will be omitted for similar processing to the work instruction processing when a thaw experiment is performed as illustrated in Fig. 7 and to the work instruction processing when a maintenance subculturing or an expansion subculturing experiment is performed as illustrated in Fig. 11.

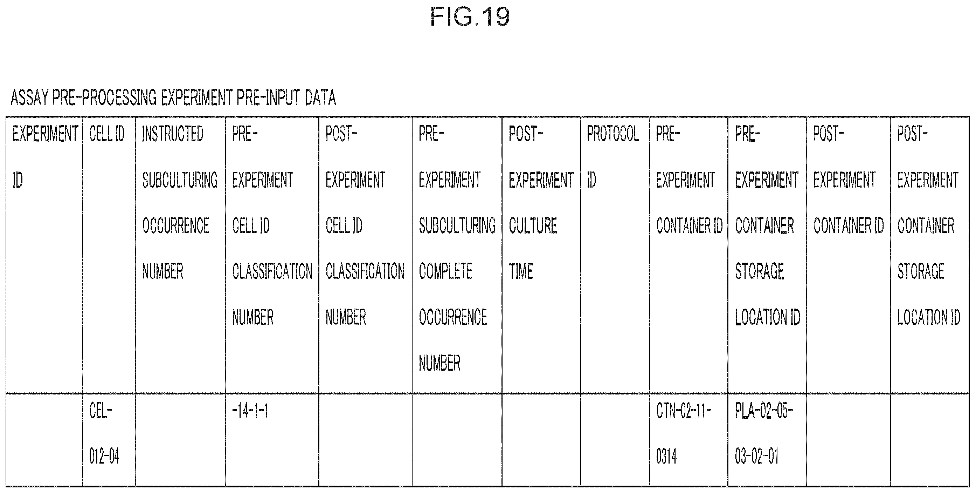

At step S30, the instruction creation section 144 creates experiment definition data for an assay pre-processing experiment (assay pre-processing experiment start-time data) from assay pre-processing experiment pre-input data. As illustrated in Fig. 19, the assay pre-processing experiment pre-input data is stored in the experiment definition DB 50 as the follow-on experiment data to a past thaw experiment, maintenance subculturing experiment, or expansion subculturing experiment.

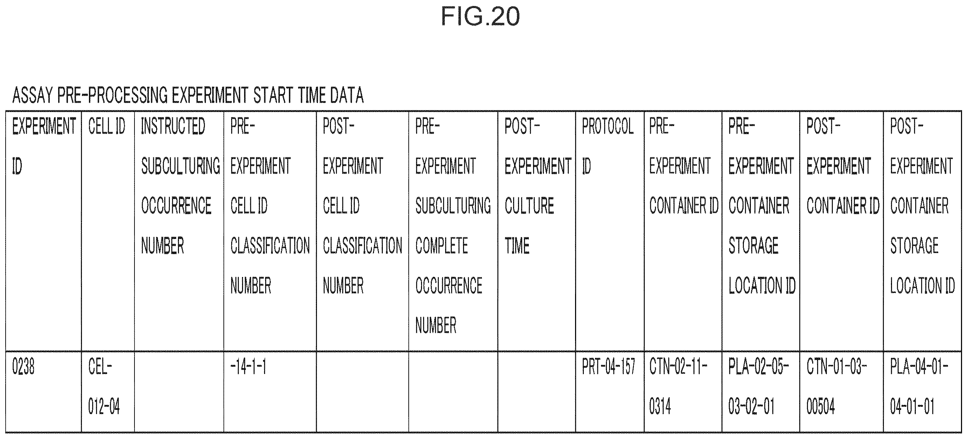

Next, at step S32, the instruction creation section 144 transmits, to the integration controller 30, a command instructing experiment execution, and experiment definition data and protocol data as illustrated in Fig. 20, and thereby instructs experiment execution.

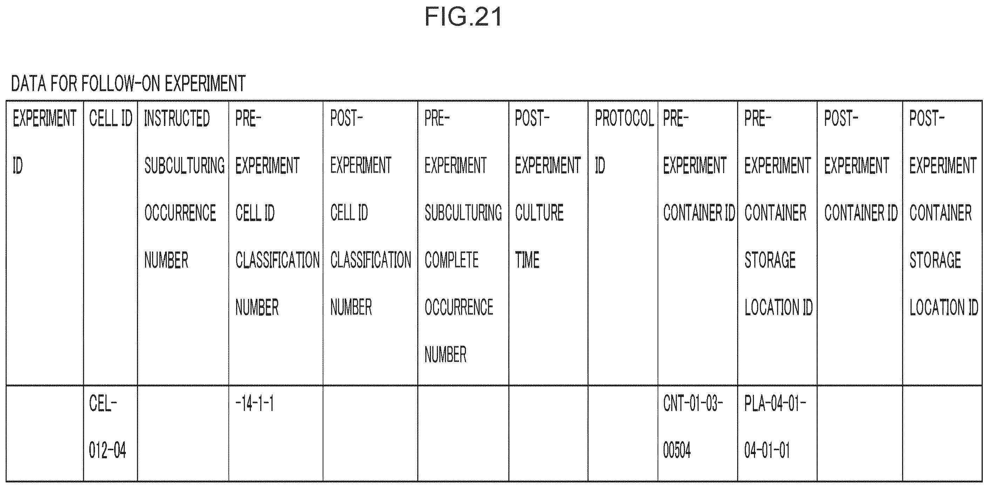

Next, at step S34, the instruction creation section 144 creates follow-on experiment data such as illustrated in Fig. 21. Then the experiment definition data access section 142 stores the created follow-on experiment data in the experiment definition DB 50. The work instruction processing for performing an assay pre-processing experiment is then ended.

Note that although explanation has been given in each of the above work instruction processing for cases in which the work instruction device 40 transmits the command instructing experiment execution, the experiment definition data, and the protocol data to the integration controller 30, there is no limitation thereto. For example, a configuration may be adopted in which the work instruction device 40 transmits the command instructing experiment execution, and an experiment ID to the integration controller 30, and the integration controller 30 acquires the experiment definition data specified by the experiment ID from the experiment definition DB 50, and acquires the protocol data specified by the protocol ID contained in the acquired experiment definition data from the protocol DB 52. A combination of the command instructing experiment execution and data relating to specification of experiment content, such as experiment definition data and the like, is configured by a preparation transport instruction, a workbench experiment instruction, or a storage transport instruction.

The integration controller 30 creates an operation command to control the mobile manipulator 10 and the workbench robot 20 based on the protocol data, and controls the mobile manipulator 10 and the workbench robot 20 via the local controller 11 and the local controller 21.

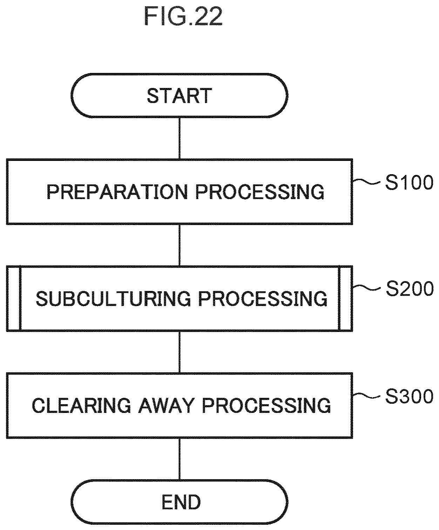

Description follows regarding control processing by the integration controller for an example of subculturing. Fig. 22 is a flowchart illustrating a flow of control processing executed by a CPU of the integration controller 30. Description follows regarding an example of adhesion culture.

At step S100, as preparation processing for subculturing, the integration controller 30 uses the mobile manipulator 10 to take a first container containing cells being cultured out from a first incubator and to transport the first container onto a workbench. The first container is, for example, a square flask or the like.

Specifically, the integration controller 30 instructs the local controller 11 of the mobile manipulator 10 to perform preparation processing. The mobile manipulator 10 then, under control of the local controller 11 receiving the instruction for preparation processing, moves to the position of the first incubator, opens the door of the first incubator, and grips the first container with the hand 13B. The mobile manipulator 10 moves the first container in a gripped state to the workbench, and arranges the first container on the workbench.

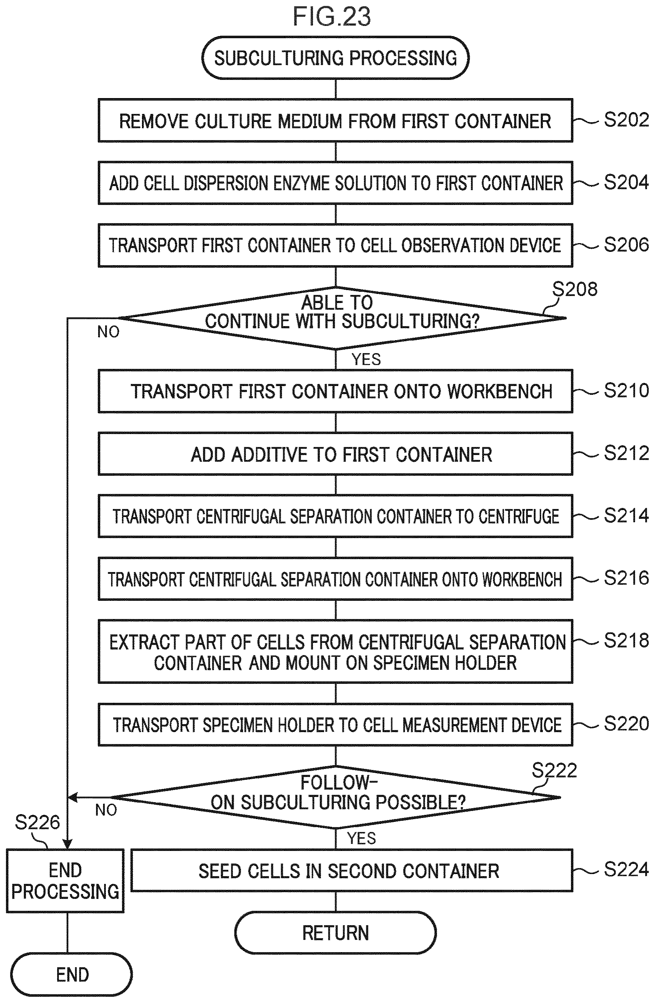

Next, at step S200, the integration controller 30 causes the mobile manipulator 10 and the workbench robot 20 to execute subculturing processing. Description follows regarding subculturing processing, with reference to Fig. 23.

At step S202, the integration controller 30 instructs the local controller 21 of the workbench robot 20 to remove a culture medium from the first container. The local controller 21 receiving the instruction then controls the workbench robot 20 so as to, for example, grip the first container with the hand 22BL, grip an aspirator with the hand 22BR, and manipulate the aspirator so as to suction and remove the culture medium inside the first container.

Next, at step S204, the integration controller 30 instructs the local controller 21 of the workbench robot 20 to add a cell dispersion enzyme solution to the first container. The cell dispersion enzyme solution is an example of a cell dispersion reagent and is, for example, trypsin. The local controller 21 receiving the instruction then controls the workbench robot 20 so as to, for example, grip an electronic pipette using the hand 22BR, manipulate the electronic pipette, and add the cell dispersion enzyme solution into the first container. The manipulation of the electronic pipette is, for example, depressing a discharge button using a finger (for example, the second finger 22B2R of the third finger 22B3R) of the hand 22BR.

Next, at step S206, the integration controller 30 instructs the local controller 11 of the mobile manipulator 10 so as to transport the first container to a cell observation device. The local controller 11 receiving the instruction then controls the mobile manipulator 10 so as to, for example, transport the first container to which the cell dispersion enzyme solution has been added on the workbench to a cell observation device such an optical microscope or the like to perform enlargement imaging acquisition and image analysis of an observation target object under computer control, and to arrange the first container at a specific position on the cell observation device. A device having a long object distance capable of observing cells through a transparent wall of the first container may be employed as the cell observation device.

Next, at step S208, the integration controller 30 determines whether or not the integration controller 30 is able to continue with subculturing based on observation results from the cell observation device. For example, the integration controller 30 acquires from the cell observation device an image of cells inside the first container or an image analysis result as the observation result, and determines that proceeding with subculturing is possible when the cells are sufficiently separated from the container walls and are in a suspended state. Note that observation of the degree of separation of the cells by the cell observation device may be performed automatically using image processing of the cell observation device or the integration controller 30, or may be performed with manual intervention. Processing transitions to step S210 in cases in which proceeding with subculturing is possible, and processing transitions to step S226 when proceeding is not possible. Note that instead of transitioning to step S226, observation may be re-attempted by the cell observation device after waiting several minutes. Sometimes separation of cells from the container walls can be expected to progress with the passage of time.

At step S210, the integration controller 30 instructs the local controller 11 of the mobile manipulator 10 to transport the first container onto the workbench. The local controller 11 receiving the instruction then controls the mobile manipulator 10 so as to take out and grip the first container from the cell observation device, to transport the first container to the workbench, and to arrange the first container on the workbench. The above step S206 to step S210 may be omitted in cases in which sufficient separation of cells from the container walls can be expected without this being confirmed by a cell observation device. For example, in cases in which there are past results in which, for culturing of the same cell type under similar conditions, separation of cells from the container walls succeeded under similar conditions.

Next at step S212, the integration controller 30 instructs the local controller 21 of the workbench robot 20 so as to add, to the first container, an additive that is at least one from out of an enzyme reaction stopping solution or a new culture medium. The local controller 21 receiving the instruction then, for example, controls the workbench robot 20 so as to grip the first container with the left hand 22BL, to grip an electronic pipette with the hand 22BR, and to add the additive into the first container. In the following, the first container containing a mixture of the cells and the additive, or a third container to which the contents of the first container are moved, is called a centrifugal separation container.

Note that in cases in which the mixture of the cells and the additive are to be moved to the third container, the integration controller 30 instructs the local controller 21 of the workbench robot 20 thereof. The local controller 21 receiving the instruction then, for example, controls the workbench robot 20 so as to manipulate an electronic pipette and suction the mixture from the first container. The manipulation of the electronic pipette is, for example, by depressing a suction button with one of the fingers of the hand 22BR (for example, the second finger 22B2R or the third finger 22B3R). The local controller 21 then controls the workbench robot 20 so as to, for example, switch to holding the centrifugal separation container such as a conical tube with the hand 22BL. Furthermore, the local controller 21 controls the workbench robot 20 so as to manipulate the electronic pipette, and move the suctioned mixture over to the centrifugal separation container. The manipulation of the electronic pipette is, for example, depressing a discharge button using a finger of the hand 22BR (for example, the second finger 22B2R of the third finger 22B3R).

Note that in cases in which the first container is to be employed as is as the centrifugal separation container, a conical tube is employed from the start as the first container rather than a square flask.

Next, at step S214, the integration controller 30 instructs the local controller 11 of the mobile manipulator 10 so as to transport the centrifugal separation container to the centrifuge. The local controller 11 receiving the instruction then controls the mobile manipulator 10 so as to transport the centrifugal separation container on the workbench to the centrifuge and to place the centrifugal separation container in a specific position in the centrifuge (for example, a rotation unit thereof). When doing so, if there is a need in the specification of the centrifuge to set the centrifugal separation container in a member for setting centrifugal separation containers, the local controller 11 controls the mobile manipulator 10 so as to, after taking the member out from inside the centrifuge, then return the member to inside the centrifuge. Furthermore, the local controller 11 may control the mobile manipulator 10 so as to depress a start button of the centrifuge and start running of the centrifuge. Note that a run start manipulation of the centrifuge may be performed by the integration controller 30 transmitting a control signal to the centrifuge.

Next, at step S216, the integration controller 30 instructs the local controller 11 of the mobile manipulator 10 so as to transport the centrifugal separation container onto the workbench. The local controller 11 receiving the instruction then controls the mobile manipulator 10 so as to recover the centrifugal separation container from the centrifuge, to grip and transport the centrifugal separation container to the workbench, and to arrange the centrifugal separation container on the workbench.

Next, at step S218, the integration controller 30 instructs the local controller 21 of the workbench robot 20 so as to extract part of the cells from the centrifugal separation container and to mount them on a specimen holder. The specimen holder employed is one matching the specification of a cell measurement device. For example, the specimen holder is a slide glass for cases in which the cell measurement device is an optical microscope. The slide glass may be configured with an indentation for inserting the specimen. Sometimes, depending on the specification of the cell measurement device, some sort of container may be employed as the specimen holder.

The local controller 21 receiving the instruction then, for example, controls the workbench robot 20 so as to manipulate the electronic micro pipette and suction part of the cells from the centrifugal separation container. The local controller 21 then controls the workbench robot 20 so as to manipulate an electronic micro pipette, and discharge the suctioned cells into, for example, an indentation of a specimen holder such as a cell counting plate. The integration controller 30 may be configured so as to cause the workbench robot 20 to perform processing to stain cells of the measurement target before cell measurement.

Note that the electronic micro pipette is an instrument that enables small quantities of cells to be suctioned and discharged. A configuration may be adopted in which ON/OFF control of suctioning and discharging in the electronic micro pipette is performed using bywire electronic control through a USB cable or the like, such that ON/OFF manipulation is not performed using the hand 22B of the workbench robot 20.

Next, at step S220, the integration controller 30 instructs the local controller 11 of the mobile manipulator 10 so as to transport the specimen holder to the cell measurement device for measuring the number or density of cells. The local controller 11 receiving the instruction controls the mobile manipulator 10 so as to, for example, transport the specimen holder on the workbench to a cell measurement device such as an optical microscope for performing enlargement imaging acquisition and image analysis of the observation target object under computer control, and to set the specimen holder in a setting unit of the cell measurement device. Note that reference here to "setting unit" indicates a setting location where measurement using the cell measurement device can be performed after the specimen holder has been set there. The specimen holder set in the setting unit may be moved to the measurement location using a function of the cell measurement device, with no need to be measurable while still set in the setting unit.

Next, at step S222, the integration controller 30 determines whether or not follow-on subculturing is possible based on measurement results from the cell measurement device.

For example, follow-on subculturing is determined to be possible in cases in which the integration controller 30 acquires a number or density of cells in the specimen holder as the measurement results from the cell measurement device, and the number or density of cells is a predetermined threshold or above. Note that measurement of the number or density of cells by the cell measurement device may be performed automatically using image processing, or may be performed with manual intervention. Processing transitions to step S224 in cases in which follow-on subculturing is possible, and processing transitions to step S226 in cases in which follow-on subculturing is not possible.

At step S224, the integration controller 30 instructs the local controller 21 of the workbench robot 20 to seed cells in one or plural second containers. Note that the second container may be similar to the first container. For expansion subculturing, sometimes the second container is a container the same as the first container but of greater size, and sometimes there is an increase in the number of containers. For a container provided with plural wells (indentations), each of the wells may be considered as being a second container.

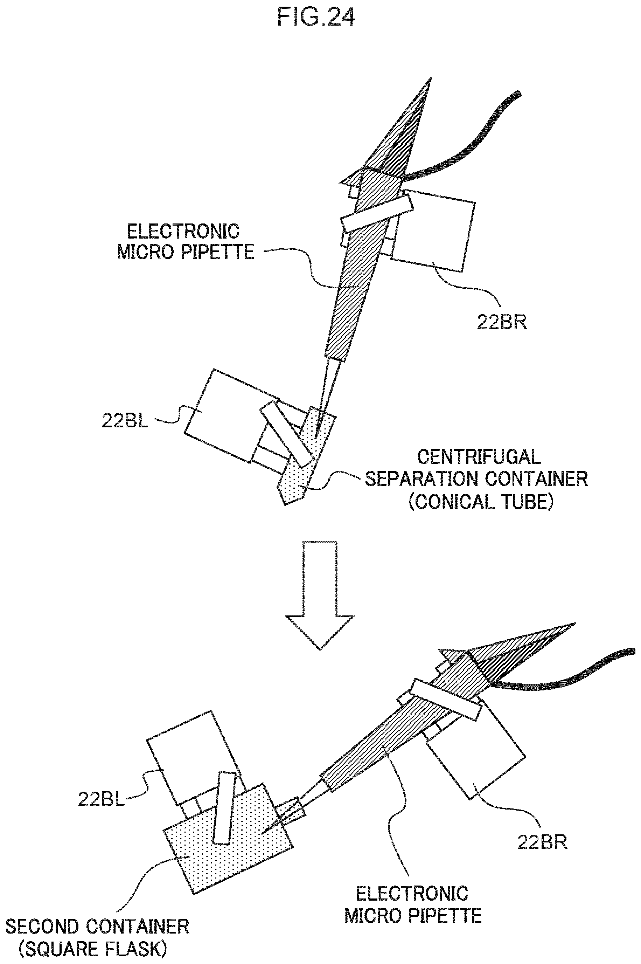

The local controller 21 receiving the instruction controls the workbench robot 20 so as to, for example as illustrated in Fig. 24, manipulate the electronic micro pipette, and to suction cells (including culture medium) of an amount for seeding the second container out from the centrifugal separation container. The local controller 21 then controls the workbench robot 20 so as to, as illustrated in Fig. 24, manipulate the electronic micro pipette and to discharge the suctioned cells into the second container that is, for example, a square flask or the like. The local controller 21 repeats such control for the number of second containers. Moreover, as required, an electronic pipette may be employed so as to augment the culture medium in the second containers.

At step S226, the integration controller 30 outputs that follow-on subculturing is not possible from the output device 45, and ends the subculturing and control processing. When doing so information indicating a reason that follow-on is not possible may be included in this output. Adopting such a configuration enables a person, or higher-level system, receiving the output to easily ascertain subculturing conditions and take appropriate measures. Note that a configuration may be adopted in which control processing is continued with the next first container inside the first incubator as the target for subculturing. In such cases processing may return to step S100 of the control processing after step S226.

Next, at step S300, the integration controller 30 causes the mobile manipulator 10 to store the second containers holding post-subculturing cells in a second incubator as clearing away processing for the subculturing. The second incubator may be the same as the first incubator.

Specifically, the integration controller 30 instructs clearing away processing to the local controller 11 of the mobile manipulator 10. Under control of the local controller 11 receiving the instruction of clearing away processing, the mobile manipulator 10 grips the second container on the workbench, moves it to the position of the second incubator, opens the door of the second incubator, and stores the second container inside the second incubator. The control processing is then ended.

As described above, the robot system according to the present exemplary embodiment includes a mobile manipulator equipped with a mechanism for moving over a floor and a mechanism for gripping an object, a workbench robot that is fixed to the workbench and equipped with a mechanism for gripping an object on the workbench, a controller for controlling operation of the mobile manipulator and a controller for controlling operation of the workbench robot, and a work instruction device that performs work instruction for executing an experiment on cells. The work instruction device includes an experiment definition data access section that accesses experiment definition data including preparation transport information that is information needed to take cells that are a target of the experiment out from a first storage location and workbench experiment specifying information that is information to specify an experiment by the workbench robot, and an instruction creation section that creates a preparation transport instruction for the controller to control operation of the mobile manipulator and a workbench experiment instruction for the controller to control operation of the workbench robot. The preparation transport instruction is an instruction created based on the preparation transport information to take cells that are an experiment target out from the first storage location and transport them to the workbench, and the workbench experiment instruction is an instruction created based on the workbench experiment specifying information to execute the experiment on the transported cells. This thereby automates an experiment handling cells that includes taking cells that are the experiment target out and, when necessary, storing cells after being processed by experiment manipulation.

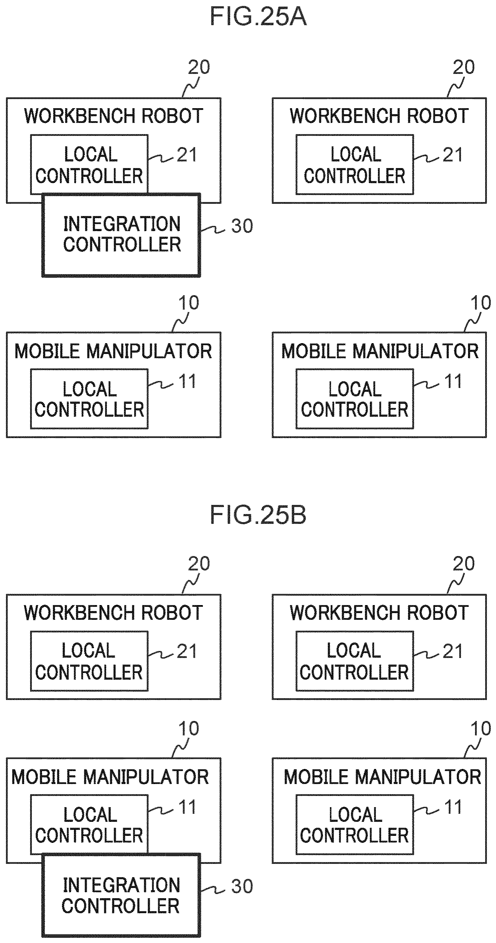

Moreover, although in the above exemplary embodiment a case has been described which, as illustrated in Fig. 2, employs the integration controller 30 independent to the mobile manipulator 10 and the workbench robot 20 as an example of a controller of the protocol data, there is no limitation thereto. For example, as illustrated in Fig. 25A, an integration controller 30 may be installed to one of the workbench robots 20 or, as illustrated in Fig. 13B, an integration controller 30 may be installed to one of the mobile manipulators 10. In such cases the work instruction device 40 may be configured capable of wireless communication with the integration controller 30.

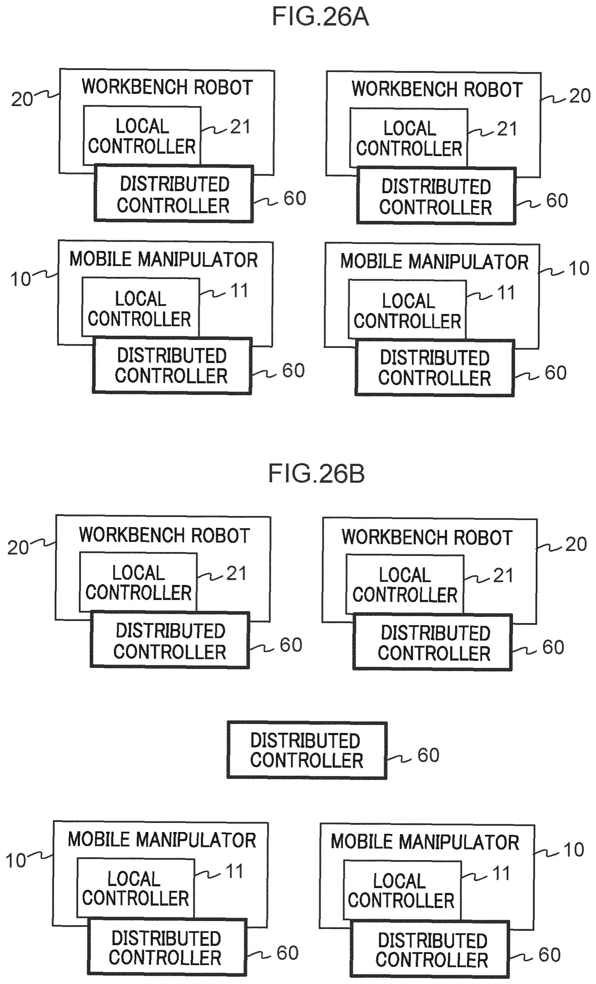

Moreover, as illustrated in Fig. 26A and Fig. 26B, the controller of the present disclosure may be configured by plural distributed controllers 60. In such cases, the distributed controllers 60 communicate with each other, and control such that the mobile manipulators 10 and the workbench robots 20 operate in coordination with each other. In such cases, the work instruction device 40 may be configured capable of wireless communication with each of the distributed controllers 60.

Note that a configuration may be adopted in which the local controllers 11, 21 are not respectively provided to the mobile manipulators 10 and the workbench robots 20, and the integration controller 30 or the distributed controllers 60 control each operation of each of the motors of the mobile manipulators 10 and the workbench robots 20.

Moreover, although description has been given in the above exemplary embodiment of an example in which the experiment definition DB is a database having a data structure in which a data record is formed for each experiment ID, there is no limitation thereto. For example, as long as a database is formed such that necessary data for performing an instructed experiment can be extracted in a format such as a relational database, the data structure of the experiment definition data is not important.

Moreover, the robot system processing executed by the CPU reading in software (a program) in the above exemplary embodiment may be executed by various processors other than a CPU. Examples of such processors include programmable logic devices (PLD) that allow circuit configuration to be modified post-manufacture, such as a field-programmable gate array (FPGA), and dedicated electric circuits and the like, these being processors including a circuit configuration custom-designed to execute specific processing, such as an application specific integrated circuit (ASIC). The robot system processing may be executed by any one of these various types of processors, or may be executed by a combination of two or more of the same type or different type of processor (such as plural FPGAs, or a combination of a CPU and an FPGA). The hardware structure of these various types of processors is more specifically an electric circuit combining circuit elements such as semiconductor elements.

Moreover, although in the above exemplary embodiments an embodiment was described in which the robot system program was pre-stored (installed) on the storage device, there is no limitation thereto. The program may be provided in a format stored on a storage medium such as a CD-ROM, DVD-ROM, Blu-ray disc, USB memory, or the like. Moreover, the program may be provided in a format downloadable from an external device over a network.

The following Supplements related to the present disclosure are disclosed.

### Supplement 1

A work instruction device (40) that performs a work instruction to execute an experiment on cells, for a robot system (100) including a mobile manipulator (10) equipped with a mechanism (12A) for moving over a floor and a mechanism (13) for gripping an object, a workbench robot (20) that is fixed to a workbench and equipped with a mechanism (22) for gripping an object on the workbench, and a controller for controlling operation of the mobile manipulator and a controller (30) for controlling operation of the workbench robot. The work instruction device includes an experiment definition data access section (142) that accesses experiment definition data (50) including preparation transport information that is information needed to take the cells that are a target of the experiment out from a first storage location and workbench experiment specifying information that is information to specify an experiment by the workbench robot, and an instruction creation section (144) that creates a preparation transport instruction for the controller to control operation of the mobile manipulator and a workbench experiment instruction for the controller to control operation of the workbench robot. The preparation transport instruction is an instruction created based on the preparation transport information to take the cells that are the experiment target out from the first storage location and transport them to the workbench, and the workbench experiment instruction is an instruction created based on the workbench experiment specifying information to execute the experiment on the transported cells.

### Supplement 2

The work instruction device of Supplement 1, wherein the work instruction device further includes an input section (146) that receives input of the workbench experiment specifying information.

### Supplement 3

The work instruction device of Supplement 1 or Supplement 2, wherein the work instruction device further includes a follow-on experiment data creation section (148) that creates follow-on experiment data to be utilized in a follow-on experiment including data to specify a container holding cells or data to specify a second storage location for storing a container holding cells.

### Supplement 4

The work instruction device of any one of Supplement 1 to Supplement 3, wherein:
the experiment definition data further includes storage transport information that is information to transport cells to a second storage location after the experiment has been performed at the workbench;
the instruction creation section further creates a storage transport instruction for the controller to control operation of the mobile manipulator; and
the storage transport instruction is an instruction created based on the storage transport information to transport cells to the second storage location after the experiment has been performed on the workbench.

### Supplement 5

The work instruction device of Supplement 4, wherein the work instruction device further includes a follow-on experiment data creation section (148) that creates follow-on experiment data that is to be utilized in a follow-on experiment including data to specify a container holding cells or data to specify the second storage location.

### Explanation of Reference Numerals

- 100: robot system
- 10: mobile manipulator
- 11: local controller
- 12: trolley
- 12A: drive wheel
- 13: robot arm
- 13A: arm
- 13B: hand
- 20: workbench robot
- 21: local controller
- 22: robot arm
- 22A: arm
- 22B, 22BL, 22BR: hand
- 22B1, 22B1L, 22B1R: first finger
- 22B2, 22B2L, 22B2R: second finger
- 22B3, 22B3L, 22B3R: third finger
- 23: vision sensor
- 30: integration controller
- 40: work instruction device
- 41: CPU
- 42: memory
- 43: storage device
- 44: input device
- 45: output device
- 46: storage medium reading device
- 47: communication I/F
- 48: bus
- 142: experiment definition data access section
- 144: instruction creation section
- 146: input section
- 148: follow-on experiment data creation section
- 50: experiment definition DB
- 52: protocol DB
- 54: storage location DB
- 60: distributed controller

## Claims

1. A work instruction device that, for a robot system including a mobile manipulator equipped with a mechanism for moving over a floor and a mechanism for gripping an object, a workbench robot that is fixed to a workbench and equipped with a mechanism for gripping an object on the workbench, and a controller for controlling operation of the mobile manipulator and a controller for controlling operation of the workbench robot, is a work instruction device that performs a work instruction to execute an experiment on cells and comprises:
an experiment definition data access section that accesses experiment definition data including preparation transport information that is information needed to take the cells that are a target of the experiment out from a first storage location and workbench experiment specifying information that is information to specify an experiment by the workbench robot; and
an instruction creation section that creates a preparation transport instruction for the controller to control operation of the mobile manipulator and a workbench experiment instruction for the controller to control operation of the workbench robot, wherein
the preparation transport instruction is an instruction created based on the preparation transport information to take the cells that are the experiment target out from the first storage location and transport them to the workbench, and
the workbench experiment instruction is an instruction created based on the workbench experiment specifying information to execute the experiment on the transported cells.

2. The work instruction device of claim 1, wherein the work instruction device further comprises an input section that receives input of the workbench experiment specifying information.

3. The work instruction device of claim 1 or claim 2, wherein the work instruction device further comprises a follow-on experiment data creation section that creates follow-on experiment data to be utilized in a follow-on experiment including data to specify a container holding cells or data to specify a second storage location for storing a container holding cells.

4. The work instruction device of claim 1 or claim 2, wherein:
the experiment definition data further includes storage transport information that is information to transport cells to a second storage location after the experiment has been performed at the workbench;
the instruction creation section further creates a storage transport instruction for the controller to control operation of the mobile manipulator; and
the storage transport instruction is an instruction created based on the storage transport information to transport cells to the second storage location after the experiment has been performed on the workbench.

5. The work instruction device of claim 4, wherein the work instruction device further comprises a follow-on experiment data creation section that creates follow-on experiment data that is to be utilized in a follow-on experiment including data to specify a container holding cells or data to specify the second storage location.
